# EUROPEAN PATENT APPLICATION

(11) **EP 3 404 054 A1**
(43) Date of publication of application: **21.11.2018**
(21) Application number: 17738236.3
(22) Date of filing: 12.01.2017
(51) Int. Cl.: C08G 63/08, A61K 31/366, A61P 19/02

(54) **PHARMACEUTICAL COMPOSITION COMPRISING 5-DODECANOLIDE, PREPARATION THEREOF AND USE OF SAME**

(30) Priority: 12.01.2016 ES 201630015
(71) Applicant: Universitat de les Illes Balears, 07122 Palma de Mallorca (ES); Centro de Investigación Biomédica en Red, 28029 Madrid (ES)
(72) Inventor: PONS BIESCAS, Antoni, 07122 Palma de Mallorca (Baleares) (ES); SUREDA GOMILA, Antoni, 07122 Palma de Mallorca (Baleares) (ES); TUR MARÍ, Josep, Antoni, 07122 Palma de Mallorca (Baleares) (ES); MARTORELL PONS, Miquel, 07122 Palma de Mallorca (Baleares) (ES); CAPÓ FIOL, Xavier, 07122 Palma de Mallorca (Baleares) (ES)
(74) Representative: Herrero & Asociados, S.L.
(86) International application number: PCT/ES2017/070017
(87) International publication number: WO 2017/121916

(57) **Abstract**

The invention relates to a pharmaceutical composition comprising 5-dodecanolide as an active ingredient. This active ingredient is isolated from pork lard using a specific treatment. The invention, therefore, also relates to a method for obtaining the composition of the invention, and to the use thereof for therapeutic purposes and, more specifically, for treating inflammatory conditions.

## Description

### FIELD OF THE INVENTION

The present invention belongs to the field of pharmaceutical compositions with anti-inflammatory activity. More particularly it relates to a pharmaceutical composition comprising 5-dodecanolide as active ingredient. This active ingredient is isolated from pork lard using a specific treatment. The invention, therefore, also relates to a method for obtaining the composition of the invention, and to the use thereof for therapeutic purposes and, more specifically, for treating inflammatory conditions.

### BACKGROUND OF THE INVENTION

Inflammation is a tissue condition consisting of a series of molecular, cellular and vascular phenomena for defensive purposes against physical, chemical and biological insults. The basic aspects that stand out in the inflammatory condition are, firstly, targeting the response, which tends to define the fight area against the insulting agent. Secondly, the inflammatory response is immediate and predominantly nonspecific, although it may promote the subsequent development of a specific response. Thirdly, the inflammatory site attracts immune cells from nearby tissues. Inflammation causes a significant dilation of blood vessels along with an opening of their pores, increasing their permeability and allowing the passage of liquid, substances, and cells from the blood to the tissues, so that they increase in volume and temperature. The completion of the inflammation is not a passive condition, rather it can be mediated by the synthesis of several molecules derived from the oxidation of polyunsaturated fatty acids such as eicosapentaenoic and docosahexaenoic acid. Among these molecules are resolvins and hydroxy derivatives from the oxidation of long chain omega-3 fatty acids (18, 20, 22 carbons) (US Patent 2009/0180961 A1), or synthetic derivatives thereof (Patent MX 2011010827). Their composition allows them to be delivered in the form of particles and nanoparticles to terminate the inflammation (WO2012135032 (A2)).

Inflammation is already described in 3000 B.C. Egyptian papyri. Celsus (a Roman writer), during the first century AD, explained its cardinal signs: Redness, Tumour, Heat, and Pain. Then, Virchow described the fifth sign: loss of function. In 1793, the Scottish surgeon John Hunter stated that "it is not a disease but a nonspecific response that has a healthy effect on the host". In 1839-1884, Julius Cohnheim described the first microscopic findings of this guarding response: increased vascular permeability and leukocyte migration. Subsequently, Elie Metchnikoff concluded that the inflammation condition generates leukocytes and antibodies for protection against microorganisms, establishing since then the relationship INFLAMMATION + INFECTION; i.e. when bacteria enter the organism, they cause an infection and the body generates inflammation as a protection. Furthermore Sir Thomas Lewis, on the basis of simple experiments of the inflammatory response made in skin, established that "chemical substances, such as histamine, locally induced by the damage, mediate the vascular changes of inflammation". This concept underpins the important discovery of chemical mediators of inflammation and the potential use of anti-inflammatory agents.

In general, inflammation can be divided into five stages:
1- Mediators release. They are molecules, most of them of elementary structure that are released or synthesised under the action of certain stimuli. When injured, the tissues will release inflammatory mediators.

Among the mediators stand out amines such as histamine or serotonin, proteolytic enzymes, nitric oxide, pro-inflammatory cytokines (such as eosinophil chemotactic factor and neutrophil chemotactic factor), and heparin.

Other substances of lipid nature constitute a second important group of inflammation mediators. These are substances synthesised *de novo* and derived from arachidonic acid through two metabolic pathways, that of the cyclooxygenase enzyme, which determines the production of prostaglandins (PG), and that of thromboxanes, and that of lipoxygenase, which leads to the formation of leukotrienes (LT).
2- Effect of the mediators. Once released, these molecules produce vascular changes and chemotactic effects promoting the arrival of molecules and immune cells at the inflammatory site. Increased vascular permeability is produced promoting the arrival at the affected area, from the blood, of molecules and immune system cells, and pain nerve endings are stimulated.
3- Arrival of molecules and immune cells at the inflammatory site. From the chronological point of view, inflammation mediators will basically produce two effects. In a first stage, vascular changes promoting the transfer of molecules from the blood to the inflammatory site, as well as the occurrence of oedema. In a second stage, later on, the vascular changes themselves, as well as the release of chemotactic factors at the site determine the arrival of immune cells (basophils, neutrophils, macrophages, lymphocytes, and eosinophils) from the blood and the surrounding tissues.
4- Regulation of the inflammatory condition. The same as most immune responses, inflammatory phenomenon also includes a series of inhibitory mechanisms to terminate or balance the condition. Some of the mediators which produce activation, by varying their concentration or acting on different receptors, will produce inhibition, achieving, in this way, a balance or modulation of the inflammatory response. The synthesis of molecules such as resolvins from the omega-3 polyunsaturated fatty acids of membrane phospholipids is involved in this inflammation termination process.
5- Repair. Stage consisting of phenomena which will determine the total or partial repair of tissues damaged by the insulting agent or the inflammatory response itself. These processes comprise the arrival at the area of fibroblasts, which will proliferate and synthesise collagen, epithelial cell proliferation, and vessel proliferation within the wound.

To reduce the inflammatory condition, anti-inflammatory products (NSAIDs) are primarily used. Their function is based on the inhibition of the cyclooxygenase enzyme reducing prostaglandin synthesis. NSAIDs are the mainstay of treatment of chronic inflammatory diseases such as the so widespread osteoarthritis and arthritis, which, as they have no cure, require the use of drugs for the treatment of symptoms (symptomatic treatment). This means that they decrease the stiffness and pain due to their anti-inflammatory and analgesic effect, but they do not cure or modify the disease. Other molecules, lipid mediators that may contribute to the completion of inflammation have been identified and whose function, utilisation, synthesis, and mode of application have been previously patented (Patent US 2009/0180961 A1; Patent MX 2011010827; WO2012135032 (A2) - 2012-10-04).

Topical forms are semi-solid and deformable preparations to spread on the skin or mucous membranes. They consist of a base or carrier and the active ingredient or drug. The bases or carriers contain the active ingredient and provide the consistency of the presentation; their correct selection depends on the type of skin to which they are to be applied and the physicochemical properties of the drug to promote its release. The purpose of these preparations is the penetration of active ingredients through the stratum corneum of the skin with insulating function. Penetration consists of two stages, absorption, which is the passage of the drug through the skin into the cellular level. The second phase is diffusion i.e. propagation through the tissues.

In treatment by topical route the following requirements must be met: intact skin, surface and focal location of the inflammation, and, in addition, the application must be continuous. Its use is indicated in injuries such as sprains, bruises, tendonitis, etc.

In traditional culture, pork sebum has been used for treating knocks and acute inflammatory conditions but without knowing the components that may be involved in these anti-inflammatory effects.

Furthermore, there are examples in literature where lard has been used in the production of compositions with anti-inflammatory activity.

For example, document WO2009077635 relates to a composition based on olive oil, pork lard and honey from bees in proportions of 1:1:1 and its use as an anti-inflammatory, anti-oedematous and anti-erythematous composition and a tissue regenerator.

Document JP2009149599 also discloses a composition of a medicinal oil based on fats and oils of black pig fed a diet supplemented with α-tocopherol, useful in the cosmetic and nutritional treatment of atopic dermatitis, inflammation, hair loss, and eczema.

On the other hand, the object of document ES2087036 is a fast acting ointment against varicose veins, which is constituted of a mixture of camphor, pork lard and bicarbonate.

The object of US2014377370A1 is a homeopathic composition comprising animal fat, including pork lard, onion and rosemary for the treatment of joints with stiffness, pain, inflammation, cramps, injury, gout or limited range of motion.

However, none of the documents described above mention which components make the pork fat exhibit an anti-inflammatory effect.

The authors of the present invention, from a determined treatment of pork fat, have achieved identifying a series of compounds with anti-inflammatory activity. But more surprisingly they have been able to identify that by means of said treatment a compound appears, which is a compound not found in untreated pork fat and which shows very potent anti-inflammatory effect. This compound, which has been identified by the inventors, is 5-dodecanolide, which is the basis of the compositions of the present invention.

### BRIEF DESCRIPTION OF THE DRAWINGS

**Figure 1****:** Effects of the ointment on the inflammation induced with zymosan on the diameter of rat paws.
**Figure 2****:** Effects of the ointment on the inflammation induced with zymosan after 4 hours as a function of the weight of the paw.

### DETAILED DESCRIPTION OF THE INVENTION

A first object of the invention is a pharmaceutical composition comprising 5-dodecanolide as active ingredient and one or more pharmaceutically acceptable excipients or carriers.

The composition of the invention can be prepared from the compound obtained commercially or by synthesis or from a process of treatment and purification of pork lard from which the inventors have achieved isolating and identifying its potent anti-inflammatory activity.

When the 5-dodecanolide is obtained from the treatment of the pork lard, which will be described below, it is observed that, in addition to 5-dodecanolide, the treated lard has a number of other compounds that are useful to enhance the anti-inflammatory activity.

Thus, in a particular embodiment, the composition of the invention further comprises 5-dodecanolide, one or more compounds selected from octadecenamide, resolvin D1, hexadecanoic acid, 9-octadecenoic acid, 6-octadecenoic acid, 9,12-octadecadienoic acid, octadecanoic acid, 5,8,11,14-eicosatetraenoic acid, 7-hexadecenoic acid, 11,14-octadecadienoic acid, hexadecanamide.

The 5-dodecanolide can be present in the composition of the invention in a range of 2-6% of the total weight of the composition, preferably of 3-5%.

In a particular embodiment, the composition of the invention comprises octadecenamide in a percentage of 2-8% of the total weight of the composition.

In another particular embodiment, the composition of the invention comprises resolvin D1 in a percentage of 1-4% of the total weight of the composition.

In another particular embodiment, the composition of the invention comprises hexadecanoic acid in a percentage of 0.01-0.7% of the total weight of the composition.

In another particular embodiment, the composition of the invention comprises 9-octadecenoic acid, 6-octadecenoic acid, or both in a percentage of 56-65% of the total weight of the composition.

In another particular embodiment, the composition of the invention comprises 9,12-octadecadienoic acid, in a percentage of 12-15% of the total weight of the composition.

In another particular embodiment, the composition of the invention comprises octadecanoic acid in a percentage of 0.1-0.5% of the total weight of the composition.

In another particular embodiment, the composition of the invention comprises 5,8,11,14-eicosatetraenoic acid in a percentage of 5.1-6.3% of the total weight of the composition.

In another particular embodiment, the composition of the invention comprises 7-hexadecenoic acid in a percentage of 1.2-3% of the total weight of the composition.

In another particular embodiment, the composition of the invention comprises 11,14-octadecadienoic acid, in a percentage of 5.5-6.1% of the total weight of the composition.

In another particular embodiment, the composition of the invention comprises hexadecanamide acid in a percentage of 0.7-1.4% of the total weight of the composition.

A particular embodiment is represented by a composition comprising, in percentage by weight with respect to the total composition:
a) 4.34-4.63% of 5-dodecanolide
b) 0.05-0.7% of hexadecanoic acid
c) 59.4-63.7% of 9-octadecenoic acid or 6-octadecenoic acid
d) 12.5-14% of 9,12-octadecadienoic acid
e) 0.13-0.47% of octadecenoic acid
f) 5.32-6.21% of 5,8,11,14-eicosatetraenoic acid
g) 1.33-2.83% of 7-hexadecanoic acid
h) 5.67-6.00% of 11,14-octadecadienoic acid
i) 0.89-1.28% of hexadecanamide
j) 3.19-6.84% of octadecenamide
k) 1.93-3.02% of resolvin D1.

The composition of the invention further comprises one or more pharmaceutically acceptable excipients or carriers. Depending on the route of administration, one or other excipient or pharmaceutical carrier well known to a person skilled in the art can be used such as, for example, support materials, lubricants, fillers, solvents, diluents, colourants, aroma and flavour conditioners such as sugars, antioxidants, waxes or esters of fatty acids, preservatives, emulsifiers and/or binders. The selection of these auxiliary additives or carriers and the amounts to be used will depend on the mode of administration of the pharmaceutical composition.

The preferred mode of administration is by topical route and, in this sense, the preferred form of the composition of the invention includes ointments, salves, gels, creams, lotions, suspensions or emulsions. The preferred embodiment of the invention comprises the composition in ointment form.

A second object of the invention is a method for obtaining a composition according to the invention comprising:
a) Extracting the lard
b) Grinding the lard
c) Brewing the ground lard
d) Separating, by sieving, the liquid lard from the pork rinds
e) Optionally, hydroalcoholic extraction of the liquid lard

Before carrying out the first stage of the method, the dressing of the pig is carried out.

The first stage comprises extracting the lard from the pig. The lard is obtained from the belly and from the adipose tissue of the omentum region. The lean part is separated from the white fat and the fat adhering to the omentum. The pig's nutrition is important in determining the amount and composition of fat accumulated by the animal.

In a second step, the lard formed by the white fat and omentum fat is ground by mechanical means, such as, for example, in a meat grinder with a chopping disc with large holes (diameter of about 2 cm). Once ground, but not too much, the fat and omentum fat are mixed until a homogeneous mass is obtained. Approximately 20 kg of mass per animal is obtained.

The ground lard is then brewed. To do this, the lard is preferably introduced into a stainless steel boiler for brewing over high heat. The fat is continuously stirred during the whole brewing process with a wooden spoon, to avoid that the fat sticks to the bottom of the boiler and burns. During the brewing process the fat liquefies and subsequently reaches the boiling point (60-80 °C). The average brewing time for about 20 Kg of fat is about 50 minutes over high heat, until the pork rinds are cooked and acquire an intense golden colour, which can happen after 90 minutes of brewing. During this brewing, the anti-inflammatory ingredients are produced and released. The production of the components of anti-inflammatory nature can be increased by increasing the proportion of protein and using fat from pigs with a diet supplemented with omega-3 polyunsaturated fatty acids.

Once the lard is brewed, it is removed from the heat and left to rest in the same pan. When it has sufficiently cooled, it is passed through a sieve in order to separate the pork rinds from the liquid lard. Subsequently, the liquid lard can be used in the next step of the method or stored in glass containers where it finishes cooling and solidifies for its optional use in the hydroalcoholic extraction stage.

The hydroalcoholic extraction and purification of the active ingredients is optional but recommendable since it allows a greater concentration of the compounds with anti-inflammatory activity. To obtain the hydroalcoholic extract from pork sebum it is melted and a mixture of alcohol:water (65:35, v:v), preferably methanol:water (65:35, v:v) and a concentrated acid, preferably 1M HCl, are added. It is stirred vigorously with mechanical aid and the phases are separated through centrifugation. The anti-inflammatory ingredients are then purified by means of a solid phase extraction in which the anti-inflammatory active ingredients are adsorbed to a hydrophobic material which is washed with water, hexane and is finally dessorbed and released with methyl formiate.

The hydroalcoholic extract obtained through this method besides 5-dodecanolide also has the following compounds: octadecenamide, resolvin D1, hexadecanoic acid, 9-octadecenoic acid, 6-octadecenoic acid, 9,12-octadecadienoic acid, octadecanoic acid, 5,8,11,14-eicosatetraenoic acid, 7-hexadecenoic acid, 11,14-octadecadienoic acid, hexadecanamide

The extract obtained can be used directly as an ointment for topical application or it can be processed for its formulation as a pharmaceutical composition adapted to any route of administration as explained above.

On the other hand, it should be noted that both the treated pork sebum and the hydroalcoholic extract contain anti-inflammatory ingredients that can be used directly or be introduced into various carriers for application on inflamed areas. These application carriers can be ointments, salves, solutions, sprays, etc.

Alternatively, the composition of the invention can be prepared using 5-dodecanolide synthesised or purified from other sources other than pork sebum or a mixture thereof with one or more of the following compounds: octadecenamide, resolvin D1, hexadecanoic acid, 9-octadecenoic acid, 6-octadecenoic acid, 9,12-octadecadienoic acid, octadecanoic acid, 5,8,11,14-eicosatetraenoic acid, 7-hexadecenoic acid, 11,14-octadecadienoic acid, hexadecanamide.

A final object of the present invention is represented by the use of the composition of the invention.

In general, the composition of the invention is applicable in the treatment of inflammation.

More specifically, the composition of the invention can be used for the treatment of inflammation associated with joint diseases such as arthritis or osteoarthritis. Therefore, since it has been proven effective in reducing the inflammatory condition initiated by fragments of the cell walls of microorganisms such as *E. coli,* it can also be used for the treatment of inflammation associated with infectious diseases such as that which occurs in mastitis that obstructs a lactiferous duct caused by bacterial infections capable of making biofilms during breast-feeding. Likewise, it can reduce the inflammation associated with acne, which is also a consequence of bacterial activity.

In any case, the composition of the invention is preferably used by topical route for the treatment of inflammation either in the form of ointment, salve, gel, cream, lotion, suspension or emulsion, and therefore, the composition of the invention is especially useful in the treatment of superficial inflammations such as traumas, sprains, contusions or tendonitis.

### EXAMPLES

The present invention is best illustrated by the following examples, which are not intended to limit its scope. Thus, for example, the specific concentrations and the nature of the ingredients and additives described in the examples can be extended to others and to other concentrations. The breed of pig used to obtain the product as well as its fattening process can vary and are not limiting to the invention. The machinery and instruments used in the examples may vary and are not limiting to the invention.

### EXAMPLE 1

In this example, an embodiment of how both the treated pork sebum and the hydroalcoholic extraction from this extract are obtained, is illustrated.

Firstly, the dressing of the pig was carried out.

Subsequently, the lard was obtained from the belly and from the adipose tissue of the omentum region. The lean part is separated from the white fat and the fat adhering to the omentum.

The pork lard formed by the white fat and omentum fat were ground in a meat grinder with a chopping disc with large holes (diameter of about 2 cm). Once ground, but not too much, the fat and omentum fat were mixed until a homogeneous mass was obtained. Approximately 20 kg of mass per animal was obtained.

Subsequently, the ground lard was subjected to brewing in a stainless steel boiler for brewing over high heat. The fat was continuously stirred during the whole brewing process with a wooden spoon, to avoid that the fat sticks to the bottom of the boiler and burns. During the brewing process the fat liquefies and subsequently reaches boiling point (60-80 °C). The lard was brewed for 90 minutes, by which time the pork rinds were brewed and acquired an intense golden colour.

Once the lard was brewed, it was removed from the heat and left to rest in the same pan. When it was sufficiently cooled, it was passed through a sieve in order to separate the pork rinds from the liquid lard. The lard, still being liquid, was stored in glass containers where it finished cooling and solidified. Part of this lard was then used in the rest of the examples and another part was subjected to hydroalcoholic extraction.

This extraction was performed as follows. 1 g of pork sebum was melted at 50-60 °C and, subsequently, 5 mL of methanol:water (65:35, v:v) and 50 µL of 1M HCl were added. It was stirred vigorously with the aid of a vortex for 1 minute, and then the phases were separated by centrifugation at 3,000 rpm for 5 min. The aqueous phase was passed through a C-18 type column (Sep-Pak® Vac 12cc (2g)), and the column was cleaned with 10 mL of water, 6 mL of hexane and finally eluted with 8 mL of methyl formiate. The eluate was dried under a stream of nitrogen at 55 °C and a hydroalcoholic extract of the pork sebum in solid state was obtained.

### EXAMPLE 2

In this example, an experiment was developed that demonstrates the anti-inflammatory properties of the ointment (pork sebum). To do this, an animal model was used in which acute inflammation was induced, and after which the anti-inflammatory product was tested.

*Experimental procedure.* To carry out the experiment, Sprague rats were used weighing 350-400 grams. The rats were divided into two groups (n = 6): a first control group that was subjected to inflammation, and a second group which, after the inflammation, was treated with the anti-inflammatory ointment.

The animals were anaesthetised with sodium pentobarbital. The inflammation was induced by a subplantar injection in one of the hind paws with zymosan (0.1 mL in saline at a concentration of 10 mg/mL). Zymosan consists of fragments of yeast cell wall (*Saccharomyces cerevisiae*) and its application generates an acute inflammation, generating a model for the study of anti-inflammatory substances. The other paw acted as a negative control and 0.1 mL of saline was injected into it. By means of this treatment, a manifest inflammation is obtained in the plantar region with a maximum of oedema at the time of injection.

One hour after the injection, a layer of 0.5 g of petroleum jelly is spread on the animals of the first group acting as control, while a layer of 0.5 g of the ointment is spread on the animals of the second group. Both products are applied uniformly so that one layer is covering the entire inflamed area.

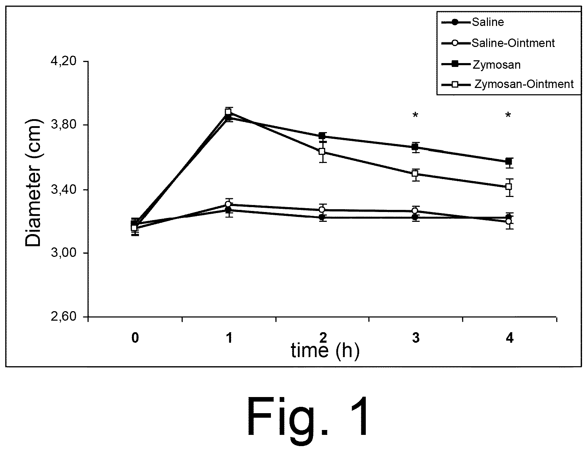

The diameter of the paw is measured at time 0 hour (before inducing inflammation), time 1 hour (before applying the ointment or petroleum jelly), and time 2, 3 and 4 hours (see Figure 1).

The diameter of the paw is influenced by the treatment to which it is subjected. The diameter at time 0 hour is similar in all animals. One hour after the injection, a differential response is observed between the saline groups (the diameter is practically unchanged) and the zymosan groups (where the increase in diameter due to inflammation is evident). At times 3 and 4 hours, it is observed how the group treated with the ointment recovers from inflammation significantly faster than the group treated with petroleum jelly. The ointment acts effectively reducing the external symptoms of inflammation.

### EXAMPLE 3

This example illustrates how the application of the ointment in the inflamed area reduces some of the biochemical mediators involved in the inflammatory condition.

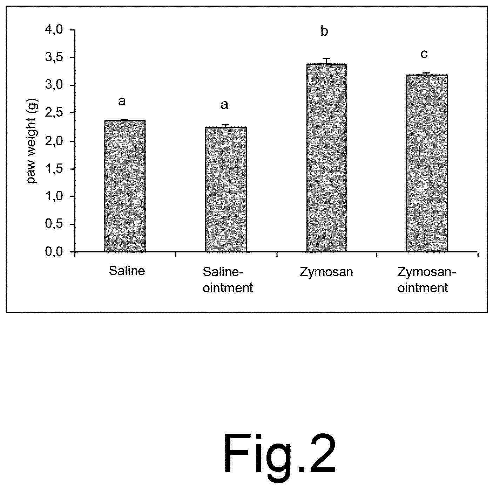

The experimental procedure is the same as that carried out in experiment 2. At 4 hours after the inflammation was generated, the animals were sacrificed by decapitation, the subplantar tissue was removed, which was weighed (see Figure 2) and then processed and blood samples were collected.

The tissue was homogenised in 50 mM phosphate buffer, pH 7.0 containing 0.5% hexadecyltrimethylammonium bromide. The homogenates were centrifuged at 8,000 g at 4 °C and the supernatant was collected. In the supernatant, enzymatic activities of antioxidant enzymes (catalase, and glutathione peroxidase) and myeloperoxidase activity (enzyme that is exclusively expressed in neutrophils and is indicative of inflammation) were determined. Nitrite levels (indirect indicator of nitric oxide production) and levels of malondialdehyde (marker of damage to lipids) and of carbonyl groups in proteins (protein damage marker) were also determined.

Neutrophils were purified from the blood. 5 mL of blood were mixed with 5 mL of PBS pH 7.4 and introduced on 4 mL of Ficoll. The samples were centrifuged at 900 g for 30 minutes at 18 °C. The supernatant was removed and 10 mL of 5% dextran was added to the precipitates. The samples were allowed to stand for 30 minutes at room temperature and finally centrifuged at 750 g, 10 minutes at 4 °C, obtaining a precipitate with the neutrophils. In the neutrophils the activities of the antioxidant enzymes and myeloperoxidase were determined.

*Enzymatic determinations.* The determinations were made in a Shimadzu UV-2100 spectrophotometer at 37 °C.

Catalase activity was determined by a method based on the decomposition of H₂O₂. This decomposition is monitored spectrophotometrically at 240 nm. The final concentration of H₂O₂ was 10 mM. The appropriate volume of sample was introduced, and was adjusted with phosphate buffer to 2 mL. The reaction was started by adding 1 mL of H₂O₂. The final volume of the assay was 3 mL.

Glutathione peroxidase activity (GPX) requires H₂O₂ as substrate and the enzymatic reaction continued to be coupled catalyzed by the glutathione reductase. The final concentrations of glutathione reductase, NADPH and H₂O₂ were respectively 0.24 U/mL, 200 □M, and 265□□M. The total volume of the reaction was adjusted to 1 mL with 200 mM phosphate buffer with 1 mM EDTA and pH 7. The absorbance was monitored at 339 nm for 3 minutes.

Myeloperoxidase activity (MPO) monitors the oxidation of guaiacol. The reaction mixture contained pH 7 phosphate buffer and 13.5 mM of guaiacol. The reaction was started with the addition of H₂O₂ 300□□M, monitoring the changes at 470 nm.

*Determination of nitrite levels.* Nitrite levels were determined as an indirect marker of nitric oxide, using a 96-well microplate colorimetric method.

The determinations were made in erythrocytes, lymphocytes, neutrophils and plasma. The samples were deproteinised by adding 1.5 volumes of acetone, and left overnight at -20 °C. They were centrifuged for 10 minutes at 15,000 g and at 4 °C, and the supernatants were collected. For the assay, 100 □l of sample were introduced into each well. Later, □□□□l of 2% w/v sulfanilamide solution in 5% HCI were added, and then 50□□l of a solution of 0.1% w/v N-(1-Naphthyl) ethylenediamine in water were added. The plates were shaken and read at 540 nm, after 30 minutes of incubation. The determinations were made in duplicate. For the calculations, a straight-line pattern with sodium nitrite was made.

*Determination of MDA levels.* The concentration of MDA was determined by using a colorimetric kit (Calbiochem) following the manufacturer's instructions.

*Determination of carbonyl groups.* To carry out the determinations, the samples were precipitated with 30% trichloroacetic acid (TCA). The precipitates were resuspended with 500□□l of a 10 mM solution of 2,4-dinitrophenylhydrazine (DNPH), for 60 minutes at 37 °C. Then the samples were precipitated with 500 □l of 20% TCA, centrifuged for 10 minutes at 1,000 g at 4 °C, and the supernatant was discarded. The precipitate obtained was washed twice with 1 mL of ethanol-ethyl acetate (1:1; v/v) to remove the DNPH that remained free, and centrifuged for 10 minutes at 1,000 g at 4 °C. The final precipitate was resuspended in 1 mL of 6 M guanidine in 2 mM phosphate buffer and pH 2.3. The samples were incubated for 40 minutes at 37 °C. Finally, the samples were centrifuged for 5 minutes at 3,000 g at 4 °C to clarify the supernatant. The concentration of carbonyl groups was determined at 360 nm, where the DNPH presents the maximum absorption.

All the results obtained in the plantar tissue were corrected by the levels of protein determined using a commercial kit (Biorad), while those obtained in neutrophils are expressed corrected by the DNA levels determined by fluorimetry.

The results relating to the effect of the ointment on the zymosan-induced inflammation on the weight of the paw are shown in Figure 2.

After 4 hours subsequent to inducing the inflammation, the weight of the paw presents similar values between the two saline groups. Treatment with the ointment significantly reduces inflammation as is evidenced by a lower paw weight.

Table 1 shows the results related to enzymatic activities, markers of oxidative damage and nitrite levels in the plantar tissue.

**Table 1. Enzymatic activities, markers of oxidative damage and nitrite levels in the plantar tissue**

| | **Saline** | **Saline ointment** | ***Zymosan*** | **Zymosan ointment** |
|---|---|---|---|---|
| **Enzymatic activities** | | | | |
| **MPO nKat/mg prot.** | 2.79 ± 0.30 | 3.02 ± 0.28 | 21.8 ± 2.4^{a} | 17.8 ± 3.0^{a} |
| | | | | |
| **GPx nKat/mg prot.** | 0.29 ± 0,02 | 0.32 ± 0.04 | 0.38 ± 0.02 | 0.36 ± 0.06 |
| **Catalase mK/mg prot.** | 11.3 ± 2.8 | 12.9 ± 1.2 | 9.17 ± 1.0 | 10.7 ± 2.2 |

| ***Oxidative damage*** | | | | |
|---|---|---|---|---|
| ***MDA* nmol/g prot** | 523 ± 79 | 542 ± 86 | 648 ± 48 | 591 ± 79 |
| **Carbonyl groups umol/mg prot** | 15.0 ± 1.1 | 16.7 ± 1.7 | 23.9 ± 0.9^{a} | 24.2 ± 1.1^{a} |
| **Nitrite umol/mg prot** | 5.81 ± 1.06 | 6.04 ± 0.54 | 14.1 ± 1.0^{a} | 11.6 ± 0.7^{b} |

### Different letters indicate significant differences between treatments (p<0.05, one-way ANOVA)

The activity of MPO is used as an indicator of inflammation because it is indicative of the infiltration of neutrophils in the tissue studied, as it is an enzyme that is expressed exclusively in this cell type. Treatment with zymosan produces a marked increase in MPO activity. The ointment does not reduce the MPO activity compared to the petroleum jelly group, possibly because the chemotactic signals for the neutrophils would be released at the beginning of the inflammation condition, before adding the ointment.

No significant differences were observed between the different treatments in the activities of the antioxidant enzymes.

The existence of oxidative damage induced by zymosan is only evident in the case of the carbonyl groups, without differences between the group treated with the ointment and the group treated with petroleum jelly.

Nitrite is used as a marker for the production of nitric oxide. Nitric oxide is an important mediator in the inflammatory condition. Treatment with zymosan significantly increases nitrite levels, which are reduced by the action of the ointment. This reduction would be involved in a faster recovery from the inflammatory condition.

Table 2 shows the results of the enzymatic activity in circulating neutrophils.

**Table 2. Enzymatic activities in circulating neutrophils**

| | **Saline** | **Saline ointment** | **Zymosan** | **Zymosan ointment** |
|---|---|---|---|---|
| **MPO nKat/ug DNA** | 4.47 ± 0.82^{a} | 4.74 ± 0.44^{a} | 7.73 ± 1,03^{b} | 4.44 ± 0.67^{a} |
| **GPx nKat/ug DNA** | 0.80 ± 0.06 | 0.79 ± 0.06 | 0.87 ± 0.0 | 0.64 ± 0.04 |
| **Catalase K/mg DNA** | 0.15 ± 0.02^{a} | 0.14 ± 0.01^{a} | 0.27 ± 0,06^{b} | 0.16 ± 0.03^{a} |

### Different letters indicate significant differences between treatments (p<0.05, one-way ANOVA)

The group treated with zymosan and petroleum jelly presents significantly higher MPO and catalase activities than the group treated with zymosan and the ointment. In fact, the group treated with the ointment presents activities similar to the two groups treated with saline. The application of the ointment reduces the degree of activation of circulating neutrophils and, therefore, would reduce the risk of suffering oxidative damage induced by the action of the neutrophils themselves.

### EXAMPLE 4

In this example, an experiment is developed that demonstrates the anti-inflammatory properties of the hydroalcoholic extract from pork sebum. To do this, a cellular model (PBMCs, human peripheral blood mononuclear cells and human neutrophils) was used in which an acute inflammation condition was simulated by contacting the cells with a component of the bacterial walls of lipopolysaccharide (LPS) nature such as *Escherichia coli.* The establishment of the inflammatory condition was tested by quantifying the production of inflammatory cytokines such as TNF-□, and after which the effect of the hydroalcoholic extract as an anti-inflammatory product was tested.

The cells were isolated from blood from the antecubital vein of 8 individuals by puncture with vacutainers with EDTA as an anticoagulant. The fractions of PBMCs and neutrophils were purified using a previously described procedure (Boyum 1964; Sureda et al. 2004a). Briefly, the blood is introduced on Ficoll in a 1.5:1 proportion and centrifuged at 900 x g, at 4 °C for 30 minutes. The fraction of PBMCs is carefully extracted, the pellet, which contains neutrophils and erythrocytes, and the plasma phase are kept, discarding the intermediate phase of Ficoll. The fraction of PBMCs is washed twice with phosphate buffer (PBS), pH 7.4, and centrifuged at 1000 g, at 4 °C for 10 minutes, finally obtaining the pellet of PBMCs.

The pellet containing erythrocytes and neutrophils is incubated at 4 °C with 0.15 M ammonium chloride to hemolyse the erythrocytes. The suspension is centrifuged at 750 g, at 4 °C for 15 minutes and the supernatant discarded. The neutrophil pellet is first washed with 0.15 M ammonium chloride and subsequently with PBS. The last wash with PBS, both that of the PBMCS pellet and the neutrophil pellet, is carried out with a 10 mL volume. This volume is distributed equally in 5 aliquots of 2 mL in five tubes so that in each tube there is the same number of cells. The tubes with the 2mL aliquots are centrifuged at 1,000 g at 4 °C for 10 minutes, the supernatant is discarded and a precipitate of cells is obtained which is used to demonstrate the inflammatory effects of the LPS and the anti-inflammatory effects of the hydroalcoholic extract from pork sebum.

The PBMCs and neutrophils are resuspended in 2 mL of RPMI-1640 culture medium (Sigma-Aldrich, Spain) alone (control) or with RPMI-1640 culture medium containing the different additives for carrying out the experimentation. The different cell cultures are incubated at 37 °C for 2 hours. At the end of the incubation, they are centrifuged at 1,000 g, at 4 °C for 10 minutes, the supernatants are collected and stored at -80 °C for the subsequent analysis of inflammatory markers such as TNF-α.

In this experiment, the following media/additives were tested 8 times with cells from 8 donors in a final volume of 2 mL:
- Control culture: containing the culture medium and cells.
- LPS Group: containing the culture medium with LPS 1 µg/mL as an additive and the cells (lipopolysaccharides from *Escherichia coli* 0127:B8; Sigma-Aldrich, Spain).
- Extract Group: containing the culture medium with a hydroalcoholic extract from pork sebum at a concentration of 1 mg/mL and the cells.
- Extract Group + LPS: containing the culture medium with a hydroalcoholic extract from pork sebum at a concentration of 1 mg/mL and the cells to which LPS (1 µg/mL) is added after 30 minutes of incubation.
- LPS Group + Extract: containing the culture medium with LPS 1 µg/mL and the cells to which a hydroalcoholic extract from pork sebum at a concentration of 1 mg/mL is added after 30 minutes of incubation.

In the supernatants of the incubation media, the following compounds are determined: TNF-α, IL-6, IL-8.

The analyses were carried out using the following ELISA kits:
- TNF-α (Diaclone, France, intra-assay coefficient of variation 3.3%, inter-assay coefficient of variation 9.0%)
- IL-6 (Diaclone, France, intra-assay coefficient of variation 4.4%, inter-assay coefficient of variation 9.1%)
- IL-8 (RayBio, intra-essay coefficient of variation 10%, inter-essay coefficient of variation 12%)

The differences between the results were analysed statistically by one-way analysis of the variance and a post hoc DMS, p<0.05, using the statistical program SPSS, version 21.0.

The results (see Table 3) are expressed as the production rate of the different parameters referred to the cells that produce them (amount/10³ cel x mL/h).

**Table 3. Production rate**

| **PBMCs** | | | | | |
|---|---|---|---|---|---|
| | Control | SPL | Extract | Extract + LPS | LPS + Extract |
| TNF-α (pg/10³ cel x mL/h) | 148 ± 11a | 390 ± 32b | 74.5 ± 26.7c | 106 ± 8a,c | 133 ± 18a, c |
| IL-6 (pg/10³ cel x mL/h) | 28.1 ± 3.2a | 39.2 ± 5.4b | 2.71 ± 1.19c | 21.1 ± 5.9a | 2.93 ± 0.57c |
| IL-8 | | | | | |
| (pg/10³ cel x mL/h) | 857 ± 82a | 1296 ± 162b | 243 ± 16c | 433 ± 66c | 186 ± 71c |
| PGE-1 | | | | | |
| (pg/10³ cel x mL/h) | 62.6 ± 20.5a | 41.6 ± 6,a | 1646 ± 307b | 2007 ± 385b | 2005 ± 394b |

| **Neutrophils** | | | | | |
|---|---|---|---|---|---|
| IL-6 (10³ cel x mL/h) | 5.70 ± 0.69a | 9.66 ± 2,45b | 4.24 ± 0.56a | 3.89 ± 0.49a | 4.91 ± 0.78a |

### Different letters indicate significant differences between groups, p<0.05.

The presence of LPS increases the production rate of TNF-α, IL-6, IL-8 by the PBMCs, and the production rate of IL-6 in the case of the neutrophils. It is shown that the LPS produces an inflammatory effect in PBMCs and in neutrophils, probably through activation and translocation to the nucleus of NF□B that allows the expression of inflammatory genes such as TNF-□, IL-6, and IL-8. The addition of the hydroalcoholic extract from pork sebum already reduces the production rate of TNF-□, IL-6 and IL-8 of the PBMCs control culture and reduces much more the production rate of these inflammatory factors due to the PBMCs activated with LPS, revealing a potent anti-inflammatory effect of the hydroalcoholic extract from pork sebum. The order in which the hydroalcoholic extract from pork sebum is introduced, before or after the LPS, does not seem to influence the anti-inflammatory response of the hydroalcoholic extract. Thus, these results are differentiated from the anti-inflammatory mechanism which some oxidation products from natural phospholipids have, which only act if, prior to the addition of the inflammatory stimulus, they are already present in the medium.

Likewise, the hydroalcoholic extract from pork sebum significantly increases the production of PGE-1 by the PBMCs without the presence of LPS modifying the production rate. It has been indicated that PGE-1 has anti-inflammatory effects. The results obtained show that either the hydroalcoholic extract has high levels of PGE1 or a precursor of its synthesis, such as has 9,12-octadecadienoic acid.

### EXAMPLE 5

This example illustrates the different composition of the pork sebum and the hydroalcoholic extract from pork sebum with respect to the composition of the starting material for the production of the pork sebum following the method described in the present invention. The components with anti-inflammatory activity are present in the already treated pork sebum and in the hydroalcoholic extract, but they are not present in the starting material for the production of the sebum or they are in lower concentrations, although some precursor components of anti-inflammatory compounds are present in lower concentration in the starting material.

To determine the starting composition of the pork sebum from the belly, an extraction of the total lipid content was carried out with organic solvents, they were derivatised by trans-esterification/esterification to the methyl esters of the fatty acids, these methyl esters and other non methylated products were separated and quantified by gas chromatography. Likewise, the content of resolvin in the dry organic extract of the sebum and the belly was quantified by immunological procedures. The components of the hydroalcoholic extract from pork sebum and belly were also identified and quantified. The hydroalcoholic extract components were trans-esterified/esterified and separated by gas chromatography coupled to a mass spectrophotometer. The identification of the different chromatographic peaks is carried out by comparison with the mass spectra of pure substances and by the retention time of the chromatographic peaks. In some cases, standards of the pure products were used to confirm their identification.

The procedures followed are summarised below. The lipid content of the sebum and the belly previously ground (5 g) is extracted following a modification of the Folch method (Folch et al., 1957) using chloroform/methanol (2:1, v:v) with 0.01% hydroxybutylanisole (BHA) as an antioxidant and 2 µL of n-heptadecanoic acid (15 mM) as internal standard. The resulting organic phase is evaporated under a stream of nitrogen at 55 °C. The dry residue is dissolved in 100 µL of hexane and, subsequently, 25 µL of derivatisation reagent (Meth-Prep™ II) is added, allowing it to react for 30 minutes at room temperature. An aliquot of 1 µL is injected into the gas chromatograph using helium as a mobile phase with a flow of 2.17 mL/min, measured at 150 °C at the top of the column. The gas chromatograph is an Agilent model 5890 (Agilent Technologies, Santa Clara, CA, USA) with a flame ionization detector (FID) and a Supelcowax® 10 Capillary and GC column, 30 mx 0.53 mm, df 0.50 µm (Supelco, Bellefonte, PA, USA). The temperature ramp starts at 150 °C with a temperature gradient of 4 °C/min up to 260 °C and then an isothermal temperature maintained for 15 minutes. The injector is at 280 °C and the FID at 300 °C.

The dry residue of the hydoralcoholic extracts from pork sebum or the pork belly obtained according to the described method from 10 g is dissolved in 100 µL of derivatisation reagent (Meth-Prep™ II) and allowed to react for 30 minutes at room temperature. An aliquot of 5 µL is injected into the gas chromatograph with helium as a mobile phase with a flow of 0.5 mL/min, measured at 150 °C at the top of the column. The gas chromatograph Agilent model 6890 (Agilent Technologies, Santa Clara, CA, USA) is coupled to an electronic impact mass detector Agilent Model 5975 (Agilent Technologies, Santa Clara, CA, USA). The chromatographic column is a Supelcowax® 10 Capillary GC column, 30 mx 0.53 mm, df 0.50 µm (Supelco, Bellefonte, PA, USA). The temperature ramp starts at 150 °C with a temperature gradient of 4 °C/min up to 260 °C and then an isothermal temperature maintained for 15 minutes. The injector is at 280 °C.

Determination of resolvin D1 is carried out on the hydroalcoholic extract of 1 g of pork sebum or belly, through the method described above, using an ELISA kit for the determination of resolvin D1 (Cayman, USA, intra-assay coefficient of variation 11.4%), following the instructions and with the interferences described therein.

The results referring to the composition of the pork sebum and pork belly, taking into account the components determined in the organic extract and those determined in the hydroalcoholic extract as well as those determined with the immunological analysis are detailed below:

**Table 4. Composition of the starting processed sebum and belly**

| Molecule (TR, min) | Sebum (%) | Belly (%) |
|---|---|---|
| C12: 0 (6.213) | 0.937 ± 0.064 | 0.674 ± 0.119 |
| C14: 0 (9.826) | 0.380 ± 0.065 | 0.465 ± 0.210 |
| C16: 0 (13.785) | 10.2 ± 1.27 | 4.30 ± 0.09 |
| C16: 1 (14.390) | 3.18 ± 0.09 | 2.70 ± 0.94 |
| C18: 0 (18.048) | 13.9 ± 1.1 | 5.43 ± 1.19 |
| C18:1n9 (18.540) | 15.6 ± 2.5 | 8.84 ± 0.51 |
| C18:2n6 (19.577) | 3.99 ± 0.429 | 4.09 ± 0.47 |
| C18:3n6 (20.254) | 0.784 ± 0.158 | 0.283 ± 0.070 |
| C18:3n3 (20.982) | 1.67 ± 0.34 | 1.61 ± 0.08 |
| C20: 0 (22.306) | 0.372 ± 0.044 | 0.204 ± 0.057 |
| C20:1n9 (22.789) | 2.81 ± 0.24 | 10.4 ± 3.1 |
| C20: 2 (23.826) | 0.784 ± 0.185 | 0.920 ± 0.153 |
| C20: 3 (24.409) | 0.150 ± 0.033 | 0.385 ± 0.071 |
| C20:4n6 (24.942) | 4.92 ± 2.26 | 2.18 ± 0.63 |
| C20:5n3 (25.212) | 0.414 ± 0.128 | 0.211 ± 0.017 |
| C22: 0 (26.398) | 2.78 ± 0.79 | 6.38 ± 0.31 |
| C22:1n9 (26.897) | 0.634 ± 0.145 | 1.39 ± 0.09 |
| C22: 2 (27.918) | 1.97 ± 0.27 | 0.565 ± 0.028 |
| C24: 0 (30.603) | 4.75 ± 1.00 | 6.24 ± 0.31 |
| C24:1n9 (31.244) | 2.45 ± 0.46 | 4.40 ± 0.57 |
| C22:6n3 (31.437) | 6.80 ± 1.63 | 11.4 ± 0.2 |
| SFA | 33.3 ± 0.8 | 23.7 ± 1.3 |
| MUFA | 24.6 ± 2.5 | 27.7 ± 1.5 |
| PUFA | 21.5 ± 3.1 | 21.7 ± 1.5 |
| 5-Dodecanolide | 0.00543 ± 0.00018 | ND |
| Hexadecanamide | 0.00129 ± 0.00025 | ND |
| Octadecenamide | 0.00607 ± 0.00060 | ND |
| Resolvin D1 (pg/g sebum) | 2360 ± 234 | 509 ± 20 |
| H₂O (%) | 0.657 ± 0.078 | |
| Identified area | 79.4 ± 2.5 | 73.04 ± 1.5 |
| Non identified area | 20.6 ± 2.1 | 26.96 ± 1.05 |
| | | |

| | | |
|---|---|---|
| ND Not detected. C20:3 = C20:3n6 + C20:3n3. SFA saturated fatty acids, MUFA monounsaturated fatty acids, PUFA polyunsaturated fatty acids. | | |

The fatty acid profile of pork sebum and pork belly is different, showing the effects of preparation of the sebum on the composition of fatty acids. It is noteworthy that the preparation of the sebum produces new components such as 5-dodecanolide, hexadecanamide and octadecenamide, as well as the release of fatty acids such as hexadecanoic acid, 9-octadecenoic acid or 6-octadecenoic acid, 9,12-octadecadienoic acid, octadecanoic acid, 5,8,11,14-eicosatetraenoic acid, 7-hexadecenoic acid, 11,14-octadecadienoic acid, which appear in the composition of the hydroalcoholic extract and which, in the composition of sebum and pork belly, are included in the respective global values of each of these fatty acids.

The composition of the hydroalcoholic extract from pork sebum is indicated in the following Table 5. In the hydroalcoholic extract from belly, these components were not detected with the exception of resolvin D1, which was detected both in the hydroalcoholic extract from sebum (2360 ± 234 pg/g sebum) and in that from belly (509 ± 20 pg/g belly).

**Table 5: GC-MASSES OF THE HYDROALCOHOLIC EXTRACT COMPOSITION FROM PORK SEBUM**

| Molecule (Certainty,%) | Molecular formula | TR (min) | Percentage (%) | | | Min-Max (%) |
|---|---|---|---|---|---|---|
| Hexadecanoic, methyl ester (93%) | C₁₇H₃₄O₂ | 4.788 | 0.603 | ± | 0.096 | 0.505 - 0.701 |
| 9-Octadecenoic, methyl ester (99%) 6-Octadecenoic, methyl ester (99%) | C₁₉H₃₆O₂ | 5.632 | 61.6 | ± | 2.2 | 59.4 - 63.7 |
| 9,12-Octadecadienoic, methyl ester (99%) | C₁₉H₃₄O₂ | 6.367 | 13.3 | ± | 0.8 | 12.5 - 14.0 |
| Octadecanoic, methyl ester (85%) | C₁₉H₃₈O₂ | 7.107 | 0.307 | ± | 0.166 | 0.136 - 0.478 |
| 5,8,11,14-eicosatetraenoic, ethyl ester (90%) | C₂₂H₃₆O₂ | 10.929 | 5.77 | ± | 0.42 | 5.32 - 6.21 |
| 5-Dodecanolide (81%) | C₁₂H₂₂O₂ | 12.935 | 4.49 | ± | 0.15 | 4.34 - 4.63 |
| 7-hexadecenoic, methyl ester (89%) | C₁₇H₃₂O₂ | 13.176 | 2.08 | ± | 0.72 | 1.33 - 2.83 |
| 11,14-octadecadienoic, methyl ester (93%) | C₁₉H₃₄O₂ | 16.956 | 5.84 | ± | 0.15 | 5.67 - 6.00 |
| Hexadecanamide (92%) | C₁₆H₃₃NO | 18.513 | 1.07 | ± | 0.21 | 0.890 - 1.28 |
| Octadecenamide (82%) | C₁₈H₃₅NO | 22.261 | 5.02 | ± | 0.50 | 3.19 - 6.84 |
| Resolvin D1 (µg/g of extract) | C₂₂H₃₂O₅ | | 2.86 | ± | 0.28 | 1.93-3.02 |

The identification of the components of the hydroalcoholic extract was carried out by mass spectrometry, taking into account the retention time of the compound. The table lists the methylated derivatives of the hydroalcoholic extract compounds, which are those separated by gas chromatography. Likewise, there are compounds present that have not been methylated and that are separated in their native form from the hydroalcoholic extract. The percentage of similarity of the mass spectrum of the chromatographic peak corresponding to each retention time with that of the pure reference compound is indicated in brackets. In the case of the 5-dodecanolide and the oleamide, standard compounds were used to verify their elution at the same retention time and their mass spectrum. The results of the mass spectra are shown below: the chromatographic peaks at the retention times of 12.935 minutes and 22.261 minutes separated from the hydroalcoholic extract have a mass spectrum that includes the mass spectra corresponding to the 5-dodecanolide and the oleamide; in turn, these pure products have the same retention times.

Some of these components of the hydroalcoholic extracts from pork sebum have anti-inflammatory activity.

### EXAMPLE 6

In this example, an experiment is developed that demonstrates the anti-inflammatory properties of some of the compounds of the hydroalcoholic extract from pork sebum. Pure components of the hydroalcoholic extract from pork sebum obtained from commercial companies of chemical products were used. A cellular model (human neutrophils) was used in which an acute inflammation condition was simulated by contacting the cells with a component of the bacterial walls of lipopolysaccharide (LPS) nature such as *Escherichia coli.* The establishment of the inflammatory process was tested by quantifying the release of catalase and myeloperoxidase to the culture medium as indicators of the degranulation of neutrophils activated by LPS. The effect of 5-dodecanolide, resolvin D1, octadecenamide and the hydroalcoholic extract from pork sebum were tested. The pure products of resolvin D1, oleamide and 5-dodecanolide come from Quimigen S.L., Spain. Neutrophils were obtained from venous blood of six donors, following the protocol described in Example 4. The hydroalcoholic extract from pork sebum was obtained following the method described in this patent. The catalase and myeloperoxidase enzymatic activities were determined following the procedures described in Example 2 in the supernatants from the centrifugation of the cultures of neutrophils incubated according to the conditions described in Example 3. The concentrations of the different components of the hydroalcoholic extract tested correspond to those provided by the hydroalcoholic extract in the culture medium.

The neutrophils of each participant were distributed among the following types of cultures:
- Control Group: culture medium RPMI-1640 (Sigma-Aldrich, Spain).
- LPS Group: culture medium with LPS of *Escherichia coli* 0127:B8 (Sigma-Aldrich, Spain) at a final concentration of 1 µg/mL.
- LPS Group with resolvin: culture medium with LPS (1 µg/mL) and Resolvin D1 (1 ng/mL).
- LPS Group with oleamide: culture medium with LPS (1 µg/mL) and Oleamide (0.06 mg/mL).
- LPS Group with 5-dodecanolide: culture medium with LPS (1 µg/mL) and 5-dodecanolide (0.04 mg/mL).
- LPS Group with Hydroalcoholic extract: culture medium with LPS (1 µg/mL) and hydroalcoholic extract (1 ng/mL).

**Table 6:Catalase (CAT) and myeloperoxidase (MPO) activity in the supernatants from incubations of neutrophils in the presence of various components of the hydroalcoholic extract from pork sebum**

| | CAT (K/10⁶ neutrophils) | CAT (%) | MPO (nKat/10⁶ neutrophils) | MPO (%) |
|---|---|---|---|---|
| Control | 53.7 ± 7.2a,c | 100 ± 13.3 a,c | 69.1 ± 39.8 a | 100 ± 57.6a |
| LPS | 116 ± 19 b | 215 ± 35.7b | 177 ± 62.0 b | 197 ± 86.3b |
| LPS + Resolvin | 75.2 ± 2.2 b,c | 139 ± 4.04 b,c | 7.38 ± 2.03 c | 10.7 ± 2.93c |
| LPS ± Oleamide | 34.3 ± 13.8 a,c | 63.7 ± 25.7a,c | 10.9 ± 3.01c | 15.8 ± 4.35c |
| LPS ± 5-Dodecanolide | 51.3 ± 14.2 a,c | 95.5 ± 26.5 a,c | 11.7 ± 1.95 c | 16.9 ± 2.81c |
| LPS + Extract sebum | 20.8 ± 7.4 a | 38.7 ± 13.8a | 8.67 ± 1.92 c | 12.5 ± 2.77c |

| | | | | |
|---|---|---|---|---|
| Statistical analysis: one-way ANOVA, post hoc DMS, p < 0.05 | | | | |

The presence of LPS induces an inflammatory response in neutrophils, promoting degranulation and a significant increase in catalase and myeloperoxidase activity in the extracellular environment. The hydroalcoholic extract from pork sebum significantly reduces the inflammatory condition induced by the LPS to values even lower than the control itself without activating with LPS. The addition of resolvin D1, 5-dodecanolide, and oleamide reduces the extracellular catalase activity to the control level while the extracellular myeloperoxidase activity is reduced to the level of the hydroalcoholic extract from the sebum of the LPS-activated neutrophil cultures.

### EXAMPLE 7

In this example, an experiment is developed that demonstrates the anti-inflammatory properties of some of the compounds of the hydroalcoholic extract from pork sebum. One of the components of the hydroalcoholic extract from pork sebum, 5-dodecanolide coming from Quimigen S.L., Spain, is used. A cellular model (human neutrophils) was used in which an acute inflammation condition was simulated by contacting the cells with a component of the bacterial walls of lipopolysaccharide (LPS) nature such as *Escherichia coli.* The establishment of the inflammatory process was tested by quantifying the production of the pro-inflammatory cytokine, the tumour necrotic factor alpha (TNF-□□, and after which the effect of 5-dodecanolide, one of the components of the hydroalcoholic extract from pork sebum was tested at different concentrations. Neutrophils were obtained from venous blood of nine donors, following the protocol described in Example 4. The levels of TNF-□ are determined in the supernatants from the centrifugation of the cultures of neutrophils incubated according to the procedures and conditions described in Example 3.

**Table 7: TNF-α levels in the supernatants from incubations of neutrophils in the presence of LPS with different concentrations of 5-dodecanolide**

| | TNF-α (pg /10⁵ neutrophils-mL/h) |
|---|---|
| Control | 15.4 ± 3.05 a,c |
| LPS | 79.1 ± 11.0 b |
| LPS + 5-dodecanolide (0.1 mg/mL) | 9.15 ± 1.53 a |
| LPS + 5-dodecanolide (0.06 mg/mL) | 13.5 ± 2.15 a,c |
| LPS + 5-dodecanolide (0.01 mg/mL) | 25.9 ± 3.78 c |
| 5-dodecanolide (0.1 mg/mL) | 7.08 ± 0.73 a |
| 5-dodecanolide (0.06 mg/mL) | 6.87 ± 0.75 a |
| 5-dodecanolide (0.01 mg/mL) | 8.2 ± 1.60 a |

| | |
|---|---|
| Statistical analysis: one-way ANOVA, post hoc DMS, p< 0.05 | |

LPS triggers an inflammatory response by significantly activating the synthesis of TNF-α which increases about five times. 5-dodecanolide does not alter the production of TNF-α control; however, it completely eliminates the pro-inflammatory effect of LPS in a manner dependent on the concentration of 5-Dodecanolide. The anti-inflammatory effect of 5-dodecanolide, measured as the lower capacity of TNF-α production by neutrophils activated with LPS is about 3 times greater if the concentration of 5-dodecanolide is 0.1mg/mL than if it is 0.01mg/mL, although all the tested concentrations reduce the rate of production of TNF-α by LPS-activated neutrophils to the control level.

## Claims

1. A pharmaceutical composition comprising 5-dodecanolide as active ingredient and one or more pharmaceutically acceptable excipients or carriers.

2. The composition according to claim 1 further comprising one or more compounds selected from octadecenamide, resolvin D1, hexadecanoic acid, 9-octadecenoic acid, 6-octadecenoic acid, 9,12-octadecadienoic acid, octadecanoic acid, 5,8,11,14-eicosatetraenoic acid, 7-hexadecenoic acid, 11,14-octadecadienoic acid, hexadecanamide.

3. The composition according to any one of the preceding claims wherein 5-dodecanolide is in a percentage of 2-6% of the total weight of the composition.

4. The composition according to any one of the preceding claims comprising, in percentage by weight with respect to the total composition:
a) 4.34-4.63% of 5-dodecanolide
b) 0.05-0.7% of hexadecanoic acid
c) 59.4-63.7% of 9-octadecenoic acid or 6-octadecenoic acid
d) 12,5-14% of 9,12-octadecadienoic acid
e) 0.13-0.47% of octadecenoic acid
f) 5.32-6.21% of 5,8,11,14-eicosatetraenoic acid
g) 1.33-2.83% of 7-hexadecanoic acid
h) 5.67-6.00% of 11,14-octadecadienoic acid
i) 0.89-1.28% of hexadecanamide
j) 3.19-6.84% of octadecenamide
k) 1.93-3.02% of resolvin D1.

5. The composition according to any one of the preceding claims in the form of an ointment for topical application.

6. A method for obtaining a composition according to any one of the preceding claims comprising:
a) Extracting the pork lard
b) Grinding the lard
c) Brewing the ground lard
d) Separating, by sieving, the liquid lard from the pork rinds
e) Optionally, hydroalcoholic extraction of the liquid lard

7. The method according to claim 6 wherein the lard is extracted from the belly and adipose tissue of the omentum.

8. The method according to any one of claims 6 or 7 wherein the grinding is done by mechanical means until a homogeneous mass is achieved.

9. The method according to any one of claims 6 to 8 wherein the brewing involves boiling the fat of the lard for a time of 50 to 90 minutes.

10. The method according to any one of claims 6 to 9 wherein the hydroalcoholic extraction is made in a 65:35 (v:v) alcohol:water solution, preferably methanol:water, and optionally, in the presence of an acid, preferably HCI.

11. The composition according to any one of claims 1 to 5 for use in the treatment of inflammation.

12. The composition for use according to claim 11 wherein the inflammation treated is inflammation associated with joint diseases such as arthritis or osteoarthritis.

13. The composition for use according to claim 11 wherein the inflammation treated is inflammation associated with infectious diseases such as mastitis or acne.

14. The composition for use according to claim 11 wherein the treatment of the inflammation is made by topical route.

15. The composition for use according to claim 13 wherein the treatment by topical route allows the treatment of superficial inflammations such as trauma, sprains, contusions, tendonitis, mastitis or acne.
